(19) Europäisches Patentamt
European Patent Office
Office européen des brevets

(11) EP 0 713 882 B1

(12) **EUROPÄISCHE PATENTSCHRIFT**

(45) Veröffentlichungstag und Bekanntmachung des Hinweises auf die Patenterteilung:
**30.12.1998 Patentblatt 1998/53**

(51) Int Cl.[6]: **C08B 37/08**, C11D 3/22, A61K 7/48, D06L 3/02

(21) Anmeldenummer: **95810713.8**

(22) Anmeldetag: **15.11.1995**

(54) **Phosphonomethylierte Chitosane**

Phosphonomethylated chitosans

Chitosanes phosphonométhylées

(84) Benannte Vertragsstaaten:
**BE CH DE ES FR GB IT LI**

(30) Priorität: **24.11.1994 CH 3544/94**

(43) Veröffentlichungstag der Anmeldung:
**29.05.1996 Patentblatt 1996/22**

(73) Patentinhaber: **Ciba Specialty Chemicals Holding Inc.**
**4057 Basel (CH)**

(72) Erfinder:
- **Kuhn, Martin, Dr.**
  **CH-4143 Dornach (CH)**
- **Maier, Thomas, Dr.**
  **D-79418 Schliengen (DE)**
- **Stehlin, Albert**
  **F-68300 Rosenau (FR)**

(56) Entgegenhaltungen:
**DE-A- 2 222 733**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 713 882 B1

Printed by Jouve, 75001 PARIS (FR)

**Beschreibung**

Die vorliegende Erfindung betrifft phosphonomethylierte Chitosane, die Herstellung dieser Verbindungen, die Verwendung dieser Verbindungen als Sequestriermittel, Komplexbildner oder Stabilisator für Bleichmittel, eine Bleichflotte, enthaltend die erfindungsgemässen phosphonomethylierten Chitosane, ein Verfahren zum Bleichen von cellulosehaltigen Fasermaterialien, das nach dem Bleichverfahren behandelte Fasermaterial, ein Verfahren zur Verhinderung von Haftung und/oder Bildung von festen Belägen auf anorganischen oder organischen Substraten, die Verwendung der erfindungsgemässen Chitosane zur Verhinderung von Haftung und/oder Bildung von festen Belägen auf anorganischen oder organischen Substraten sowie die Verwendung der erfindungsgemässen Chitosane als Befeuchtungsmittel der Haut und von Schleimhäuten.

Die Phosphonomethylierung ist bekannt, z.B. aus J. Org. Chem. 31, 1503-1607 (1966). Sie verläuft in Analogie zu der Mannich-Reaktion, indem man Amine, die mindestens eine NH-Gruppe aufweisen, im sauren pH-Bereich mit phosphoriger Säure und Formaldehyd umsetzt. Aus der >N-H- Gruppe des Amins entsteht dabei die Gruppe der Formel

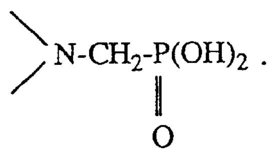

Produkte mit derartigen Gruppen werden beispielsweise als Komplexbildner für mehrwertige Metallionen verwendet.

Der vorliegenden Erfindung liegt die Aufgabe zugrunde, neue phosphonomethylierte Verbindungen zur Verfügung zu stellen, die aus leicht zugänglichen, aus der Natur stammenden Einsatzstoffen hergestellt werden können.

Die Aufgabe wird erfindungsgemäss gelöst mit phosphonomethylierten Chitosanen, die wiederkehrende Einheiten der Formel

(1) 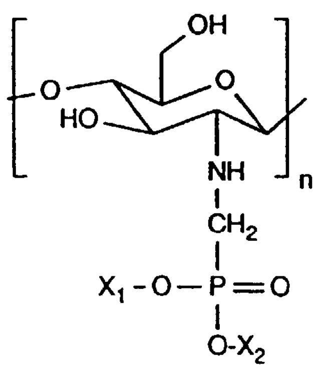

aufweisen, in Betracht. $X_1$, $X_2$ unabhängig voneinander, Wasserstoff, $C_1$-$C_5$-Alkyl oder ein Alkali- oder Ammoniumion und n 50 bis 4000 bedeuten.

Vorzugsweise kommen Verbindungen der Formel (1) in Betracht, worin

$X_1$ und $X_2$, unabhängig voneinander, Alkalimetall und
n 200 bis 2000

bedeuten.

Die Phosphonomethylierung von Chitosan erfolgt in an sich bekannter Weise durch Umsetzung einer Verbindung, enthaltend wiederkehrende Einheiten der Formel

(1a) 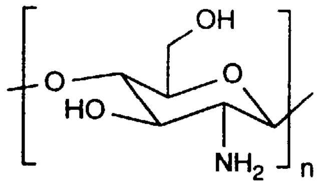

mit Formaldehyd und phosphoriger Säure in saurem, vozugsweise salzsaurem Medium. Der pH-Wert des Reaktionsgemisches beträgt dabei 0 bis 4, vorzugsweise 0 bis 2. Die Umsetzung kann besonders vorteilhaft bei Temperaturen von 80 bis 120°C durchgeführt werden. Die Reaktionszeiten reichen von 5 bis 24, vorzugsweise 10 bis 20 Stunden.

Nach Beendigung der Reaktion stellt man den pH-Wert der erhaltenen Lösung mit einer starken Base, vorzugsweise verdünnter Natronlauge, auf 6 bis 9, vorzugsweise 7 bis 8 ein. Die Reinigung der Produktlösung erfolgt z.B. durch Umkehrosmose und anschliessender Lyophilisierung des erhaltenen Konzentrats.

Die Herstellung der erfindungsgemässen phosphonomethylierten Chitosane stellt einen weiteren Erfindungsgegenstand dar.

Die erfindungsgemässen Chitosanderivate sind Polyampholyte mit filmbildenden und sequestrierenden Eigenschaften auch in Gegenwart von Erdalkalimetallionen. Auf Grund ihrer ausgeprägten sequestrierenden Wirkung für Schwermetallionen bei schon geringen Konzentrationen können die Produkte zur Entfernung solcher Kationen aus kontaminiertem Wasser verwendet werden, zum Beispiel zur Entfernung von Eisen- oder Kupferionen aus Leitungswasser. Ferner können sie als Sequestriermittel in der Nahrungsmittelindustrie, der pharmazeutischen Industrie und der Textilindustrie eingesetzt werden, sowie auch als Detergentien alleine oder in Kombination mit kationischen, anionischen oder neutralen Detergentien.

Die erfindungsgemässen Chitosanderivate weisen eine überraschend gute komplexierende Wirkung für Metallionen auf, wodurch die Ausfällung von polymeren Metallsalzen, besonders bei mehrwertigen Kationen, günstig beeinflusst oder verhindert werden kann. Ferner zeigen sie eine modulierende Wirkung bei Kristallisationsvorgängen, besonders auf die Bildung von Kristallkeimen, deren Wachstum und die Morphologie der gebildeten Kristalle und deren Grössenverteilung, sowie auf die Aggregations- und Adhäsionseigenschaften. Sie eignen sich daher zur Wasserbehandlung, um zum Beispiel die Abscheidung von Belägen in wasserführenden Systemen (Wasserbehandlungsanlagen), zum Beispiel auf Gefässwänden, Membranen oder Leitungen zu verhindern. Sie können auch zur Vorbehandlung von Textilien, zum Beispiel Baumwolle verwendet werden. Die erfindungsgemässen phosphonomethylierten Chitosane verhindern auch die Bildung von Belägen aus anorganischen und/oder organischen Bestandteilen. Sie eignen sich daher auch als Zusätze in Zahnpflegemitteln zur Verhinderung der Bildung von Zahnbelägen, sowie als Zusätze zu Waschmitteln.

Weiterhin finden die erfindungsgemässen phosphonomethylierten Chitosane Anwendung als Bleichmittelstabilisatoren, beispielsweise in Waschmitteln oder bei der Bleiche von Textilien, Zellstoff oder Papierrohstoff. Die eingesetzten Komplexbildner binden dabei die in den Bleichflotten vorhandenen Calcium-, Magnesium-, Eisen-, Kupfer- oder Manganionen und verhindern gleichzeitig die Ausfällung von Erdalkalicarbonat oder -hydroxid und die Zersetzung der Perverbindung.

Einen weiteren Erfindungsgegenstand bildet daher das Applikationsverfahren zum Bleichen von Cellulose enthaltenden Fasermaterialien. Das Verfahren ist dadurch gekennzeichnet, dass man das Fasermaterial mit einer wässrigen Flotte behandelt, welche mindestens die erfindungsgemässe phosphonomethylierte Chitosanverbindung, ein Alkalimetallhydroxid, ein wasserlösliches Magnesiumsalz und eine Perverbindung enthält.

Die wässrigen Bleichflotten zur Durchführung des entsprechenden Applikationsverfahrens, bei welchem die erfindungsgemässe phosphonomethylierte Chitosanverbidnung verwendet wird, stellen weitere Gegenstände der Erfindung dar.

Als Perverbindung kommen z.B. Alkalimetallperoxidisulfate oder vor allem Alkalimetallperoxisulfate, wobei das Kalium- und insbesondere das Natriumperoxidisulfat oder -peroxisulfat bevorzugt sind, in Betracht. Hierbei ist das Natriumperoxisulfat ($Na_2S_2O_8$) ganz besonders bevorzugt, das in der Regel als Feststoff eingesetzt wird. Als Perverbindung steht Wasserstoffperoxid im Vordergrund des Interesses, das auf Grund seiner höheren Stabilität in der Regel als konzentrierte, etwa 30 bis 60 gewichtsprozentige Lösung eingesetzt wird.

Als Alkalimetallhydroxide kommen vor allem Kalium- oder insbesondere Natriumhydroxid, vorzugsweise als konzentrierte, etwa 30 gewichtsprozentige Lösung oder als festes Ätzkali oder vor allem Ätznatron, in Betracht.

Die Bleichflotten enthalten neben der erfindungsgemässen Zusammensetzung, Alkalimetallhydroxid und einer Perverbindung als fakultative Komponenten gegebenenfalls auch Netz- bzw. Waschmittel, Entschäumungs- bzw. Entlüftungsmittel und/oder optische Aufheller.

Netz- bzw. Waschmittel werden als fakultative Komponente der Bleichflotten in der Regel eingesetzt, sofern beim

zu behandelnden cellulosehaltigen Fasermaterial die Cellulose in rohem Zustand vorliegt oder insbesondere aus roher Baumwolle besteht. Als Netz- bzw. Waschmittel kommen anionische oder nichtionogene Tenside, insbesondere aber deren Gemische, in Betracht. Bevorzugte anionische Tenside sind z.B. Alkarylmonosulfonate, Fettsäurekondensationsprodukte, Eiweissspaltprodukte oder deren Salze und vor allem Alkylmonosulfonate und Alkylbenzolmonosulfonsäuren mit 12 bis 22 Kohlenstoffatomen im Alkylrest. Bevorzugte nichtionogene Tenside sind z.B. Addukte aus Alkylenoxiden, vor allem Propylen- und insbesondere Ethylenoxid und Alkylphenolen mit z.B. 4 bis 12 Kohlenstoffatomen im Alkylrest, vor allem Fettsäureamiden und insbesondere gegebenenfalls endgruppenverschlossenen Fettalkoholen, wobei Addukte aus Ethylenoxid und Fettalkoholen besonders bevorzugt sind und als Gemisch mit den Alkylmonosulfonaten der angegebenen Art im Vordergrund des Interesses stehen. Als weitere Komponenten in diesen Gemischen eignen sich auch Silikontenside.

Entschäumungs- bzw. Entlüftungsmittel sind als fakultative Komponente der Bleichflotte vor allem bei Vorhandensein eines Netz- bzw. Waschmittels in der Bleichflotte erforderlich. Hierbei handelt es sich z.B. um höhere Alkohole, insbesondere Isooctanol, jedoch vor allem um Entschäumungs- und/oder Entlüftungsmittel auf Siliconbasis, insbesondere um Siliconölemulsionen.

Die ebenfalls als fakultative Komponente der Bleichflotten eingesetzten optischen Aufheller zur Erzielung eines besonders hohen Weissgrades der behandelten Materialien gehören im allgemeinen der Styryl- und Stilbenreihe an, wie z.B. Distyrylarylene, Diaminostilbene, Ditriazolylstilbene, Phenylbenzoxazolylstilbene, Stilbennaphthotriazole und Dibenzoxazolylstilbene. Bevorzugt sind optische Aufheller vom Typ der Distyrylbiphenyle oder der Bistriazinylaminostilbene, welche Sulfonsäuregruppen enthalten, z.B. sulfonierte Bisstyrylbiphenyl- und Bistriazinyl-Derivate, insbesondere die als Alkalimetallsalze, vor allem als Kalium- oder vorzugsweise als Natriumsalze vorliegenden Bis(Phenylamino-morpholino-s-triazinyl)-stilben-disulfonsäuren. Diese werden vorzugsweise als handelsübliche, wässrige, etwa 20 bis 30 gewichtsprozentige Flüssigformulierungen gegebenenfalls eingesetzt.

Das Applikationsverfahren zum Bleichen von Cellulose enthaltenden Fasermaterialien unter Verwendung der erfindungsgemässen Bleichmittelzusammensetzung wird nach an sich bekannten Methoden durchgeführt. Man unterscheidet hierbei zwischen einer Behandlung in langen Flotten, einem Kaltlager- und einem Unterflottenbleichverfahren.

In langen Flotten wird das Material einer Behandlung bei einem Flottenverhältnis von etwa 1:3, z.B. in einem Jigger, bis etwa 1:40, z.B. in einer Haspelkufe während etwa 1 bis 3 Stunden bei erhöhter Temperatur unterworfen, wobei die Behandlungstemperatur etwa 40 bis 140°C beträgt, vorzugsweise 60 bis 100°C unter Normalbedingungen, d.h. unter atmosphärischem Druck oder über 100°C, vorzugsweise 105 bis 140°C unter sogenannten HT-Bedingungen (HT = Hochtemperatur).

Das Unterflottenbleichverfahren erfolgt unter den gleichen Applikationsbedingungen, mit dem Unterschied, dass die Konzentration an zugesetzter Perverbindung und Alkali höher ist.

Bei dem Kaltlagerbleichverfahren wird das zu behandelnde Material durch Eintauchen in eine Klotzflotte imprägniert, wobei die Klotzflotte in der Regel eine Temperatur von 20 bis 95°C aufweist, und anschliessend abgequetscht. Vorzugsweise erfolgt die Imprägnierung bei Raumtemperatur. Die durch die Imprägnierung aufgebrachten Chemikalien wirken sodann auf das Textilmaterial ein, wobei Einwirkungszeit, gegebenenfalls erhöhte Temperatur und Chemikalien-Konzentration in direktem Zusammenhang stehen und die gewählten Bedingungen von der Beschaffenheit des Fasermaterials und vor allem der zur Verfügung stehenden Anlage abhängen. Anschliessend wird die imprägnierte und vorzugsweise aufgerollte Ware bei Raumtemperatur (15 bis 30°C), z.B. während 3 bis 24 Stunden gelagert, wobei die Kaltverweilzeit von der Art des applizierten Bleichbades abhängig ist. Anschliessend werden die Fasermaterialien in der Regel zuerst mit heissem Wasser von etwa 90 bis 98°C und dann mit warmem und zuletzt mit kaltem Wasser gründlich gespült, gegebenenfalls mit z.B. Essigsäure neutralisiert und hierauf vorzugsweise bei höheren Temperaturen bis z.B. 150°C entwässert und getrocknet.

Die wässrigen Bleichflotten haben, je nachdem, ob es sich um eine Langflotten-, Unterflotten- oder Kaltlagerbleiche handelt, die angegebenen Zusammensetzungen.

Die Langflottenbleichen enthalten

0,5 bis 5, vorzugsweise 0,5 bis 2 g/kg der wässrigen, erfindungsgemässen phosphonomethylierten Chitosanverbindung,
0,1 bis 1%, vorzugsweise 0,2 bis 0,5 % eines wasserlöslichen Magnesiumsalzes,
1 bis 6, vorzugsweise 2 bis 5 ml/kg einer Perverbindung,
1 bis 5, vorzugsweise 0,1 bis 2 g/kg eines Alkalimetallhydroxids,
0 bis 2, vorzugsweise 0,5 bis 1 g/kg eines Netz- bzw. Waschmittels,
0 bis 0,5, vorzugsweise 0,05 bis 0,5 g/kg eines Entschäumungs- bzw. Entlüftungsmittels, und
0 bis 5, vorzugsweise 1,5 bis 4 g/kg eines optischen Aufhellers.

Die Unterflottenbleichen enthalten

0,5 bis 5, vorzugsweise 0,5 bis 2 g/kg der wässrigen, erfindungsgemässen phosphonomethylierten Chitosanverbidnung,
0,5 bis 1,5 %, vorzugsweise 0,2 bis 1 % eines wasserlöslichen Magnesiumsalzes,
2 bis 12, vorzugsweise 4 bis 10 ml/kg einer Perverbindung,
1 bis 5, vorzugsweise 1,5 bis 4 g/kg eines Alkalimetallhydroxids,
0 bis 2, vorzugsweise 0,5 bis 1 g/kg eines Netz- bzw. Waschmittels,
0 bis 0,5, vorzugsweise bis 0,05 bis 0,5 g/kg eines Entschäumungs- bzw. Entlüftungsmittels, und
0 bis 30, vorzugsweise 2 bis 10 g/kg eines optischen Aufhellers.

Die Kaltlagerbleichen enthalten

5 bis 15, vorzugsweise 8 bis 12 g/kg der wässrigen, erfindungsgemässen phosphonomethylierten Chitosanverbidnung,
0,5 bis 2,5 %, vorzugsweise 1 bis 1,5 % eines wasserlöslichen Magnesiumsalzes,
30 bis 60, vorzugsweise 50 bis 60 ml/kg einer Perverbindung,
20 bis 50, vorzugsweise 25 bis 40 g/kg eines Alkalimetallhydroxids,
0 bis 2, vorzugsweise 0,05 bis 1 g/kg eines Entschäumungs- bzw. Entlüftungsmittels, und
0 bis 6, vorzugsweise 2 bis 6 g/kg eines optischen Aufhellers.

Das zu behandelnde cellulosehaltige Fasermaterial kann in verschiedenen Verarbeitungsformen vorliegen, z.B. als loses Material, Garn, Gewebe oder Gewirke. Hierbei handelt es sich in der Regel stets um textile Fasermaterialien, die aus reinen textilen Cellulosefasern oder aus Gemischen von textilen Cellulosefasern mit textilen Synthesefasern hergestellt werden.

Als cellulosehaltige Fasern kommen z.B. solche aus regenerierter Cellulose, wie z.B. Zellwolle oder Viskose, solche aus nativer Cellulose, wie z.B. Hanf, Leinen, Jute, Ramie und vor allem Baumwolle und als synthetische Fasern solche aus Polyacrylnitril und vor allem aus Polyester und Polyamid in Betracht.

Gewebe aus Baumwolle oder regenerierter Cellulose oder Mischgewebe aus Baumwolle und Polyester und aus Baumwolle und Polyamid eignen sich besonders gut dazu, erfindungsgemäss behandelt zu werden, wobei Baumwollgewebe und -gewirke im Vordergrund des Interesses stehen. Mit z.B. Tensiden vorgewaschene Materialien kommen auch in Betracht. Es ist auch möglich, geschlichtete Baumwollfasern zu bleichen, wobei das Bleichen vor oder vorzugsweise nach dem Entschlichten erfolgt.

Die unter Verwendung der erfindungsgemässen Bleichmittelzusammensetzung behandelten Fasermaterialien zeichnen sich durch ihre Schalenfreiheit, ihren niedrigen Aschengehalt und vor allem ihren hohen Weissgrad aus. Die Cellulose, bzw. der Celluloseanteil des gebleichten Materials weist zudem keine Schädigungen bzw. keinen wesentlichen Abbau des Cellulosepolymerisationsgrades (= DP-Grad, DP = Durchschnittspolymerisation) auf. Mit der erfindungsgemässen Zusammensetzung wird eine besonders hohe Stabilisierungswirkung, insbesondere in Bleichflotten, die einen pH-Wert > 11 aufweisen, erzielt. Schwermetallspuren, insbesondere Eisen(III)-Ionen, die eine spontane Zersetzung der in der Bleichflotte vorhandenen Perverbindungen bewirken können, werden wirkungsvoll gebunden bzw. inaktiviert Außerdem wird mit der erfindungsgemässen Zusammensetzung eine kristallisationshemmende Wirkung gegenüber Wasserhärte erzielt. Die Wiederbenetzbarkeit eines mit der erfindungsgemässen Zusammensetzung behandelten Fasermaterials ist ausgezeichnet. Ein weiterer wesentlicher Vorteil der erfindungsgemässen Zusammensetzung ist ihre gute biologische Abbaubarkeit.

Ein weiterer Gegenstand der Erfindung ist die Verwendung der erfindungsgemässen phosphonomethylierten Chitosane zur Verhinderung der Haftung und/oder Bildung von festen Belägen auf anorganischen oder organischen Substraten. Die Beläge, die häufig eine krustenartige Konsistenz besitzen, können sich aus anorganischen und/oder organischen Bestandteilen zusammensetzen, zum Beispiel Salzen und Polymeren auch biologischen Ursprungs. Bei den Substraten kann es sich um anorganische und/oder organische Materialien oder um biologische Materialien handeln, zum Beispiel Glas, Keramiken, Metalle und Metallegierungen, natürliche oder synthetische Kunststoffe, Papier, Textilien, Leder, oder pflanzliche oder tierische Organe oder Gewebe.

Ein weiterer Gegenstand der Erfindung ist daher auch ein Verfahren zur Verhinderung von Haftung und/oder Bildung von festen Belägen auf anorganischen oder organischen Substraten, das dadurch gekennzeichnet ist, dass man einer Flüssigkeit oder einem Mittel, das sich in Kontakt mit einem anorganischen oder organischen Substrat befindet, das erfindungsgemässe phosphonomethylierte Chitosan zusetzt, bevorzugt aber 0,01 bis 20 Gew.-%, besonders bevorzugt 0,1 bis 10 Gew.-%.

Die erfindungsgemässen phosphonomethylierten Chitosane haben filmbildende Eigenschaften. Beim Eindampfen von wässrigen Lösungen bilden sich transparente, feste und Wasser enthaltende Filme, die luft- und feuchtigkeitsdurchlässig sind. Auf Grund dieser Eigenschaft und ihrer wasserspeichernden Wirkung eignen sie sich auch als Befeuchtungsmittel für die Haut oder der Schleimhäute in kosmetischen Präparaten, wie zum Beispiel Haut- und Haarpflegemittel und Deodorantien. Die erfindungsgemässen phosphonomethylierten Chitosane sind dabei in der Lage, Eisenionen auf der Haut zu binden und können daher die in Cremes, insbesondere Sonnencremes,üblicherweise eingesetzte EDTA ersetzen.

Einen weiteren Erfindungsgegenstand bildet daher ein kosmetisches Präparat enthaltend mindestens eine Verbindung, die wiederkehrende Einheiten der Formel (1) aufweist sowie kosmetisch verträgliche Träger- oder Hilfsstoffe.

Die erfindungsgemässe kosmetische Zusammensetzung enthält z.B. 0,001 bis 15, wie z.B. 0,1 bis 15, vorzugsweise 0,001 bis 10, und ganz besonders 0,002 bis 5 Gew.-%, bezogen auf das Gesamtgewicht der Zusammensetzung, einer Verbindung, die wiederkehrende Einheiten der Formel (1) aufweist und kosmetisch verträglichen Hilfsstoffe.

Die kosmetische Zusammensetzung kann durch physikalisches Mischen einer Verbindung, die wiederkehrende Einheiten der Formel (1) aufweist, mit den Hilfsstoffen durch gewöhnliche Methoden, wie z.B. durch einfaches Zusammenrühren der zwei Materialien, erfolgen.

Die erfindungsgemässe kosmetische Zubereitung kann als Wasser-in-Öl- oder Öl-in-Wasser-Emulsion, als Öl-in-Öl-Alkohol-Lotion, als vesikulare Dispersion eines ionischen oder nichtionischen amphiphilen Lipids, als Gel, fester Stift oder als Aerosol-Formulierung formuliert werden.

Für die erfindungsgemässen kosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxyxlierter Fettalkohol; ein gegebenenfalls ethoxylierter Sorbitanester; eine ethoxylierte Fettsäure; oder ein ethoxyliertes Glycerid.

Die kosmetische Formulierung kann auch weitere Komponenten wie z.B. Emollients, Emulsionsstabilisatoren, Hautbräunungsbeschleuniger, Verdickungsmittel, Feuchtigkeit-Retentionsmittel wie z.B. Glycerin, Konservierungsmittel Duft- und Farbstoffe enthalten.

Die erfindungsgemässen phosphonomethylierten Chitosane weisen in wässrigen Lösungen auch eine viskositätssteigernde und dispergierende Wirkung auf, wodurch sie sich als Zusätze in Suspensionen, Emulsionen und wässrigen Lösungen eignen, zum Beispiel bei der Herstellung von Lebensmitteln oder Wirkstoffkonzentraten sowie Farbstoff- und Pigmentzubereitungen.

Die erfindungsgemässen phosphonomethylierten Chitosane können auch biocide Wirkungen, zum Beispiel bakteriostatische, fungistatische oder algicide Wirkungen besitzen.

In den folgenden Beispielen beziehen sich Prozent und Teile stets auf das Gewicht.

Herstellung der erfindungsgemässen phosphonomethylierten Chitosanverbindungen

Beispiel 1: Im 601-Emaillekessel werden

2,163 Teile Chitosan (Flocken),
2,23 Teile phosphorige Säure,
6,9 Teile Wasser und
7,0 Teile Salzsäure (32%ig)

bei Raumtemperatur vorgelegt. Es entsteht eine beige, flockige Suspension, die relativ gut rührbar ist. Der Ansatz wird auf 80°C erhitzt. Bei dieser Temperatur werden nun 4,27 Teile Formaldehyd (37%ig) innerhalb von 20 Minuten zugetropft. Das Gemisch wird jetzt deutlich flüssiger und brauner.

Anschliessend lässt man bei der eingestellten Temperatur 18 Stunden reagieren. Nach 2 Stunden wird der Ansatz tiefbraun. Man kühlt auf Raumtemperatur ab und stellt mit ca. 9 Teilen Natronlauge 50% auf einen pH-Wert von 7,5 ein.

Die braune Produktlösung wird in ein Fass abgefüllt und zur Umkehrosmose gegeben. Das zurückerhaltene Konzentrat wird lyophilisiert. Man erhält das Produkt mit wiederkehrenden Einheiten der Formel

(101) 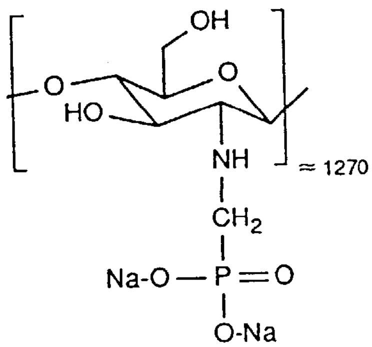

Beispiel 2: Bestimmung des Komplexier- und Dispergiervermögens

a. Bestimmung des Ca-Komplexiervermögens

1 g der Verbindung der Formel (101) wird mit ca. 80 ml deionisiertem Wasser gelöst und mit 1 N NaOH auf einen pH-Wert von 8 eingestellt. Man setzt 2 ml 10%iger Natriumcarbonatlösung als Fällungsmittel hinzu, stellt mit NaOH auf einen pH-Wert von 12 ein und ergänzt die Vorlage auf 100 ml. In die Lösung wird eine Nephelometer-Optrode eingetaucht und bei Stufe 2 (Messbereich 10 - 20'000 NTU; NTU = "Nephelometric Turbidity Units") auf 0 geeicht. Bis zur beginnenden Trübung wird in Portionen von 0,1 ml und einem Zeitintervall von einer Minute unter Rühren mit 0,5 M Calciumacetatlösung titriert. Die pH-Wert-Änderungen während der Titration werden entsprechend korrigiert

Die Berechnung des Ca-Bindevermögens erfolgt nach folgender Formel:

$$\text{mg gebundenes Ca/g Produkt} = \frac{\text{ml 0,5 M Calciumacetat x 20}}{\text{Produkteinwaage}}$$

Der für die Verbindung der Formel (101) ermittelte Wert von 168 mg/g zeigt, dass es sich bei dem erfindungsgemässen phosphonomethylierten Chitosan um eine Verbindung mit gutem Komplexiervermögen handelt.

b. Bestimmung des $CaCO_3$-Dispergiervermögens

Man verfährt wie unter a. beschrieben mit dem Unterschied, dass man anstelle von 2 ml 10%iger Natriumcarbonatlösung 10 ml 10%ige Natriumcarbonatlösung einsetzt.

Die Berechnung des $CaCO_3$-Dispergiervermögens erfolgt nach der unter a. angegebenen Formel.

Der für die Verbindung der Formel (101) ermittelte Wert von 50 mg/g zeigt, dass es sich bei dem erfindungsgemässen phosphonomethylierten Chitosan um eine Verbindung mit gutem $CaCO_3$-Dispergiervermögen handelt.

Beispiel 3: Anwendung in der Kosmetik

Herstellung einer Sonnenschutzcreme:

| | |
|---|---|
| 7,5 T | Octylmethoxyzinnamat |
| 5 T | Octylsalicylat, |
| 5 T | Methylanthranilat |
| 2 T | Isocetylalkohol |
| 2 T | Cetearylalkohol |
| 1,5 T | Glycerylstearat |

(fortgesetzt)

| | |
|---|---|
| 1 T | PEG-40-Stearat |
| 1 T | Methicon |
| 0,75 T | Cetylalkohol |
| 0,25 T | Tocopherylacetat und |
| 6 T | Zinkoxid |

werden gemischt und auf 75 bis 80°C erhitzt. Anschliessend dispergiert man mit einer handelsüblichen Kugel-, Vibrations- oder Hammermühle

| | |
|---|---|
| 0,5 T | Magnesium-aluminiumsilikat in |
| 65,8 T | Wasser. |

Der Dispersion gibt man sodann

| | |
|---|---|
| 0,5 T | Xanthan-Gummi |

und mischt, bis eine homogene Lösung entsteht. Unter Zugabe von

| | |
|---|---|
| 0,2 T | der Verbindung der Formel (101) |

wird auf 75 bis 80°C erhitzt. Zu dieser Lösung gibt man unter Rühren die anfangs gemischten und auf 75 bis 80°C erhitzten Verbindungen. Man mahlt 10 bis 15 Minuten, kühlt auf 40°C ab und gibt

| | |
|---|---|
| 1 T | Propylenglykol |

hinzu. Unter weiterem Rühren lässt man auf Raumtemperatur abkühlen.

Die Zubereitung eignet sich als feuchtigkeitsspendende Sonnenschutzcreme.

**Patentansprüche**

**1.** Phosphonomethylierte Chitosane, enthaltend wiederkehrende Einheiten der Formel

(1) 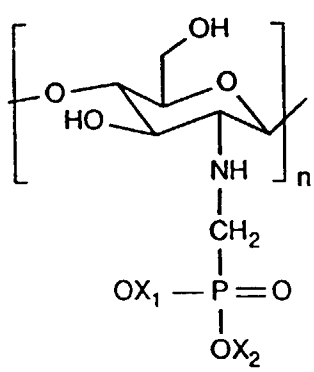 ,

worin

$X_1$ und $X_2$, unabhängig voneinander, Wasserstoff, $C_1$-$C_5$-Alkyl oder ein Alkali- oder Ammoniumion und
n 50 bis 4000

bedeuten.

**2.** Chitosane nach Anspruch 1, dadurch gekennzeichnet, dass in Formel (1)

$X_1$ und $X_2$, unabhängig voneinander, Alkalimetall und
n 200 bis 2000

bedeuten.

**3.** Verfahren zur Herstellung der Chitosane nach Anspruch 1 oder 2 dadurch gekennzeichnet, dass man eine Verbindung, enthaltend wiederkehrende Einheiten der Formel

(1a) 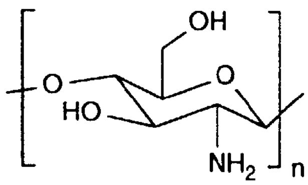

mit Formaldehyd und phosphoriger Säure, vorzugsweise in saurem Medium bei einem pH-Wert von 0 bis 4, einer Temperatur von 80 bis 120°C und einer Reaktionszeit von 5 bis 24 Stunden zur Verbindung, enthaltend wiederkehrende Einheiten der Formel (1) umsetzt, worin

n die in Formel (1) angegebene Bedeutung hat.

**4.** Verwendung der Chitosane nach Anspruch 1 als Sequestriermittel für Erdalkalimetallionen, Komplexbildner für Metallionen oder Stabilisator für Bleichmittel.

**5.** Verfahren zum Bleichen von cellulosehaltigen Fasermaterialien, dadurch gekennzeichnet, dass man das Fasermaterial mit einer wässrigen Flotte behandelt, welche mindestens eine Perverbindung, ein Alkalimetallhydroxid, ein wasserlösliches Magnesiumsalz und eine Chitosanverbindung nach Anspruch 1 enthält.

**6.** Bleichflotte enthaltend mindestens eine Perverbindung, ein Alkalimetallhydroxid, ein wasserlösliches Magnesiumsalz und eine Chitosanverbindung nach Anspruch 1.

**7.** Das nach Anspruch 5 gebleichte, cellulosehaltige Fasermaterial.

**8.** Verwendung der phosphonomethylierten Chitosane nach Anspruch 1 zur Verhinderung von Haftung und/oder Bildung von festen Belägen auf anorganischen oder organischen Substraten.

**9.** Kosmetisches Präparat enthaltend mindestens ein phosphonomethyliertes Chitosan nach Anspruch 1 sowie kosmetisch verträgliche Träger- oder Hilfsstoffe.

**10.** Verwendung der phosphonomethylierten Chitosane nach Anspruch 1 als Befeuchtungsmittel der Haut und von Schleimhäuten.

**Claims**

**1.** A phoshonomethylated chitosan comprising repeating units of formula

(1) 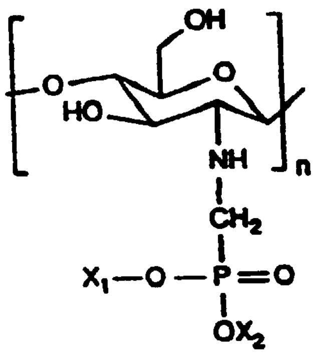,

wherein $X_1$ and $X_2$ are each independently of the other hydrogen, $C_1$-$C_5$alkyl or an alkali metal ion or ammonium ion and
n is 50 to 4000.

**2.** A chitosan according to claim 1, wherein in formula (1) $X_1$ and $X_2$ are each independently of the other alkali metal and
n is 200 to 2000.

**3.** A process for the preparation of a chitosan according to claim 1 or 2, which comprises reacting a compound comprising repeating units of formula

(1a) 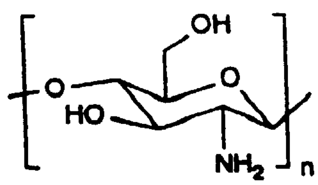

with formaldehyde and phosphorous acid, preferably in acidic medium at a pH of 0 to 4, a temperature from 80 to 120°C and in a reaction time from 5 to 24 hours, to give the compound comprising repeating units of formula (1) wherein n is as defined for formula (1).

**4.** Use of a chitosan according to claim 1 as sequestrant for alkaline earth metal ions, as complexing agent for metal ions or as stabiliser for bleaching agents.

**5.** A process for bleaching cellulosic fibre materials, which comprises treating the fibre material with an aqueous liquor which comprises at least one per compound, an alkali metal hydroxide, a water-soluble magnesium salt and a chitosan according to claim 1.

**6.** A bleaching liquor comprising at least one per compound, an alkali metal hydroxide, a water-soluble magnesium salt and a chitosan according to claim 1.

**7.** The cellulosic fibre material bleached according to claim 5.

**8.** Use of a phosphonomethylated chitosan according to claim 1 for preventing adhesion of and/or the formation of solid deposits on inorganic or organic substrates.

**9.** A cosmetic preparation comprising at least one phosphonomethylated chitosan according to claim 1 together with cosmetically acceptable carriers or adjuvants.

**10.** Use of a phosphonomethylated chitosan according to claim 1 as humectant for the skin and mucous membranes.

## Revendications

1. Chitosanes phosphonométhylés contenant des motifs répétitifs de formule

(1)

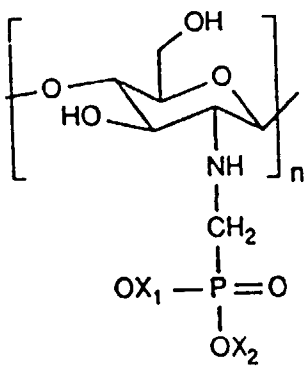

dans laquelle $X_1$ et $X_2$ représentent, indépendamment l'un de l'autre, un atome d'hydrogène, un groupe alkyle en $C_{1-5}$, ou un ion de métal alcalin ou un ion d'ammonium, et n représente un nombre compris entre 50 et 4000.

2. Chitosanes selon la revendication 1, caractérisés en ce que dans la formule (1), $X_1$ et $X_2$ représentent, indépendamment l'un de l'autre, un métal alcalin et n vaut entre 200 et 2000.

3. Procédé de fabrication des chitosanes selon la revendication 1 ou 2, caractérisé en ce que l'on fait réagir un composé contenant des motifs répétitifs de formule (1a)

(1a)

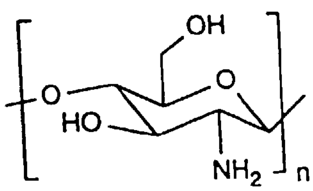

avec du formaldéhyde et avec de l'acide phosphorique de préférence dans un milieu acide, à un pH compris entre 0 et 4, à une température allant de 80 à 120 °C et pendant un temps compris entre 5 et 24 heures, pour obtenir un composé contenant des motifs répétitifs de formule (1) dans laquelle n a la signification indiquée pour la formule (1).

4. Utilisation des chitosanes selon la revendication 1 en tant qu'agents séquestrants d'ions de métaux alcalino-terreux, agents complexants les ions métalliques ou stabilisateurs d'agents de blanchiment.

5. Procédé de blanchiment de matériaux fibreux cellulosiques, caractérisé en ce que l'on traite le matériau fibreux avec un bain aqueux contenant au moins un composé peroxydé, un hydroxyde de métal alcalin, un sel de magnésium hydrosoluble et un chitosane selon la revendication 1.

6. Bain de blanchiment contenant au moins un composé peroxydé, un hydroxyle de métal alcalin, un sel de magnésium hydrosoluble et un chitosane selon la revendication 1.

7. Matériau fibreux contenant de la cellulose, blanchi selon la revendication 5.

8. Utilisation des chitosanes phosphonométhylés selon la revendication 1 pour prévenir l'adhérence et/ou la formation de dépôts solides sur des substrats minéraux ou organiques.

9. Formulation cosmétique contenant au moins un chitosane phosphonométhylé selon la revendication 1 ainsi que des véhicules ou adjuvants acceptables d'un point de vue cosmétique.

10. Utilisation des chitosanes phosphonométhylés selon la revendication 1 comme agent hydratant de la peau et des muqueuses.